(19) **Europäisches Patentamt**

**European Patent Office**

**Office européen des brevets**

(11) Veröffentlichungsnummer: **0 181 447 B1**

(12) **EUROPÄISCHE PATENTSCHRIFT**

(45) Veröffentlichungstag der Patentschrift:
03.05.89

(21) Anmeldenummer: **85110737.5**

(22) Anmeldetag: **27.08.85**

(51) Int. Cl.⁴: **A 61 M 5/14**, A 61 M 1/02, A 61 M 1/36

(54) **Gerät zum Anwärmen von Infusions- und Transfusionslösungen.**

(30) Priorität: **21.09.84 DE 3434772**

(43) Veröffentlichungstag der Anmeldung:
**21.05.86 Patentblatt 86/21**

(45) Bekanntmachung des Hinweises auf die Patenterteilung:
**03.05.89 Patentblatt 89/18**

(84) Benannte Vertragsstaaten:
**AT BE CH DE FR GB IT LI LU NL SE**

(56) Entgegenhaltungen:
**DE-A- 1 942 162**
**DE-A- 2 833 730**
**DE-B- 1 293 954**
**GB-A- 1 446 412**
**GB-A- 1 578 015**
**US-A- 3 443 060**
**US-A- 3 629 552**

(73) Patentinhaber: **Stihler Medizintechnik GmbH,**
**Julius-Hölder-Strasse 4, D-7000 Stuttgart 70 (DE)**

(72) Erfinder: **Dietrich, Kurt, Ringstrasse 15, D-7335 Salach (DE)**
Erfinder: **Stihler, Axel, Kiefernweg 5, D-7000 Stuttgart 70 (DE)**
Erfinder: **Theilacker, Wolfgang, Tailfinger Strasse 22, D-7000 Stuttgart 80 (DE)**

(74) Vertreter: **Patentanwälte Kohler - Schwindling - Späth, Hohentwielstrasse 41, D-7000 Stuttgart 1 (DE)**

## Beschreibung

Die Erfindung betrifft ein Gerät zum Anwärmen von Infusions- und Transfusionslösungen, bei dem die Infusions- oder Transfusionslösung durch einen Schlauch geführt wird, der in eine Nut eingelegt ist, die schraubenlinienförmig auf der Aussenseite eines mittels Heiz- und Regeleinrichtungen auf eine bestimmte Temperatur gebrachten Wärmeaustauschzylinders vorgesehen ist.

Ein derartiges Gerät ist bekannt (Prospekt der Fa. DIDECO s.P.a.). Dabei wird ein ca. 4 m langer Schlauch in die schraubenlinienförmig auf der Aussenseite einer sog. Wärmespirale eingebrachten Nuten mit vier Windungen eingelegt. Nachteilig an dieser Anordnung ist, dass es die erforderliche Länge dieses Schlauches bedingt, dass das normale Transfusions- und Infusionsbesteck bzw. der zu diesem Besteck gehörende Schlauch, der kürzer ist, nicht verwendet werden kann. Die Länge von ca. 4 m bei dem bekannten Gerät ist jedoch erforderlich, um die gewünschte Bluttemperatur zu erreichen. Die normalerweise bei Infusionen verwendeten Schläuche, die Bestandteile des normalen Infusionsbestecks sind, haben aber nur eine Länge von ca. 190 cm. Sie sind also mit dem bekannten Gerät nicht verwendbar. Ausserdem besteht auch die Gefahr einer ungleichmässigen Erwärmung des Blutes, da das Blut, auf der Innenseite des Schlauches fliesst, der direkten Kontakt zu dem Wärmeaustauschzylinder hat, eventuell höher erwärmt wird als das Blut, das in dem Schlauch entlang seiner Aussenseite fliesst, entlang der der Schlauch mit der atmosphärischen Umgebung direkt in Kontakt ist.

Im Vordergrund der Bemühungen um ein Gerät zum Anwärmen von Infusions- und Transfusionslösungen steht neben der Tatsache, dass die Infusionen und Transfusionen, insbesondere Blut, überhaupt angewärmt werden sollen, das Erfordernis einer genauen Regelbarkeit der verwendeten Heizeinrichtungen auf einen bestimmten Wert. Die Regelbarkeit der Heizeinrichtungen steht dabei in einem engen Zusammenhang mit der Ausbildung der Heizeinrichtungen selbst, da durch letztere die technischen Vorgaben dafür definiert sind, um bestimmte Anforderungen an die Genauigkeit der Regelung, insbesondere aber auch an die Zuverlässigkeit der Regelung, erfüllen zu können.

Während man einerseits durch die Anwärmung einer Infusion oder Transfusion Kälteschocks vermeidet, wie sie z.B. durch Unterkühlung eines Patienten leicht entstehen können (bspw. bei der Notversorgung nach einem Unfall), muss man andererseits mit absoluter Sicherheit gewährleisten, dass die Infusion oder Transfusion auf keinen Fall auf mehr als 39,5° erhöht wird, da bei höherer Temperatur die Gefahr einer Koagulation des Blutes besteht. In diesem Zusammenhang ist ein Gerät bekannt geworden (EP 0 012 123A1) bei dem, von einem Temperatursensor gesteuert, die Transfusions- oder Infusionslösung bei kleineren Strömungsgeschwindigkeiten auf einen bestimmten Strömungspfad umgeleitet wird. Diese Lösung geht auch von der Verwendung eines Einsatzbeutels aus und benötigt zusätzliche Umschaltelemente für die Strömung der Infusion oder Transfusion.

Der Erfindung liegt demgemäss die Aufgabe zugrunde, ein Gerät der eingangs genannten Art zu schaffen, das erheblich einfacher als die bekannten Geräte ist. Dabei stellt sich insbesondere die Aufgabe, die am Markt erhältlichen üblichen Infusionsbestecke, also ganz «normale» Infusionsbestecke verwenden zu können; diese haben normalerweise eine Schlauchlänge von 1,9 m. Die Aufgabe schliesst also ein, dass man keinen Einsatzbeutel u. dgl. mehr braucht. Es sollen keine speziell für das Anwärmgerät konzipierten Schläuche oder Leitungen erforderlich sein. Es versteht sich dabei ferner, dass jede Lösung dieser Aufgabe geeignet sein muss, bei Überschreiten einer Temperatur von 39,5 °C mit absoluter Sicherheit eine Abschaltung zu garantieren.

Erfindungsgemäss wird diese Aufgabe dadurch gelöst, dass der Wärmetauschzylinder auf seiner Aussenseite von einer Wärmeschutzmanschette umgeben ist und dass dem Gerät mindestens eine Sicherheitsabschaltung zugeordnet ist, die bei Überschreiten eines bestimmten einstellbaren Temperaturwertes des Wärmetauschzylinders die Heizung abschaltet.

Die Wärmeschutzmanschette verhindert ein Abstrahlen von Wärmeenergie von dem Schlauch in die atmosphärische Umgebung vollständig. Sie sorgt dafür, dass die Aussentemperatur die Temperatur des Schlauches und der Flüssigkeit nicht beeinflusst und dass der Schlauch auf seinem gesamten Querschnitt die gleiche Temperatur aufweist. Da die Wärmeschutzmanschette auch dazu verwendet werden kann, um den Schlauch in die Nut einzudrücken, wird hierdurch auch ein guter Wärmeübergang ohne diese behindernde Luftspalte zwischen der Wand der Nut und dem Schlauch erreicht. Da die Aussenatmosphäre nicht auf den Schlauch einwirkt, kann die Temperatur der Flüssigkeit zumindest in der Anfangsphase leichter gesteuert und konstant gehalten werden. Diese leichte Regelbarkeit auf eine konstante Temperatur erleichtert es der Sicherheitsabschaltung, bei einer bestimmten Temperatur, nämlich vorzugsweise bei 39,5°, die Heizung mit absoluter Sicherheit abzuschalten bzw. herunterzuregeln. Es ist daher weitgehend ausgeschlossen, dass die Sicherheitsschaltung zu früh oder aber zu spät reagiert. Die gute Temperaturkonstanz verhindert auch ein periodisches Auf- und Abregeln der Heizung, was in der Nähe der Temperatur, bei der die Sicherheitseinrichtung abschaltet, besonders störend in Erscheinung treten kann.

Weitere Vorteile der Erfindung liegen darin, dass es möglich ist, mit zwei oder drei Umschlingungen eines Zylinders, der vorzugsweise einen Durchmesser von 10 bis 15 cm hat, auszukommen. Damit können normale Serieninfusionsbestecke eingesetzt werden. Dies trägt in besonderem Masse zur Einfachheit der Handhabung und zur Kostensenkung bei dem Einsatz derartiger Geräte bei, wobei gleichzeitig eine grosse Zahl der eingangs genannten Schwierigkeiten vermieden werden. Es sind auch keine zusätzlichen Hilfsmittel (wie beim Einsetzen von Schläuchen in Wassertanks) notwendig. Wegen des Wegfalls des Druckverlustes durch spezielle Einsatzbeutel oder besonders lange Leitungen kann die Vorrichtung auch zusammen mit Infusionspumpen verwendet werden. Eine Infusions- oder Transfusionslösung läuft ohne Rest durch den Schlauch. Ebenso kann das Gerät zum Anwärmen von Spüllösungen bei chirurgischen Eingriffen eingesetzt werden. Ferner ist die einfache und schnelle Einsatzbereitschaft hervorzuheben, die sowohl im Operationssaal, aber auch bei der Ambulanz, der Intensiv-, Dialyse- und allen Pflegestationen, sowie im Rettungswesen (Rettungswagen, Rettungshubschrauber) gegeben ist. Besonders im Rettungswesen besteht damit eine einfache Möglichkeit, ausgekühlte Unfallpatienten mit angewärmter Infusion oder Transfusion am Unfallort und während des Transportes zu versorgen. Hervorzuheben ist die kleine kompakte Bauweise und der geringe Stromverbrauch, der die Stromversorgung durch 12 V-Systeme eines Fahrzeugs bzw. seiner Betterie ermöglicht.

Ein Ausführungsbeispiel der Erfindung und ihrer vorteilhaften Weiterbildungen werden im folgenden anhand der beigefügten Zeichnungen beschrieben. Es stellen dar:

Figur 1 ein Ausführungsbeispiel;

Figur 2 eine Ansicht des Ausführungsbeispiels in Richtung der Pfeile II–II in Fig. 1;

Figur 3 eine Draufsicht auf das Ausführungsbeispiel;

Figur 4 einen Schaltplan der Heizungsregelungseinheiten.

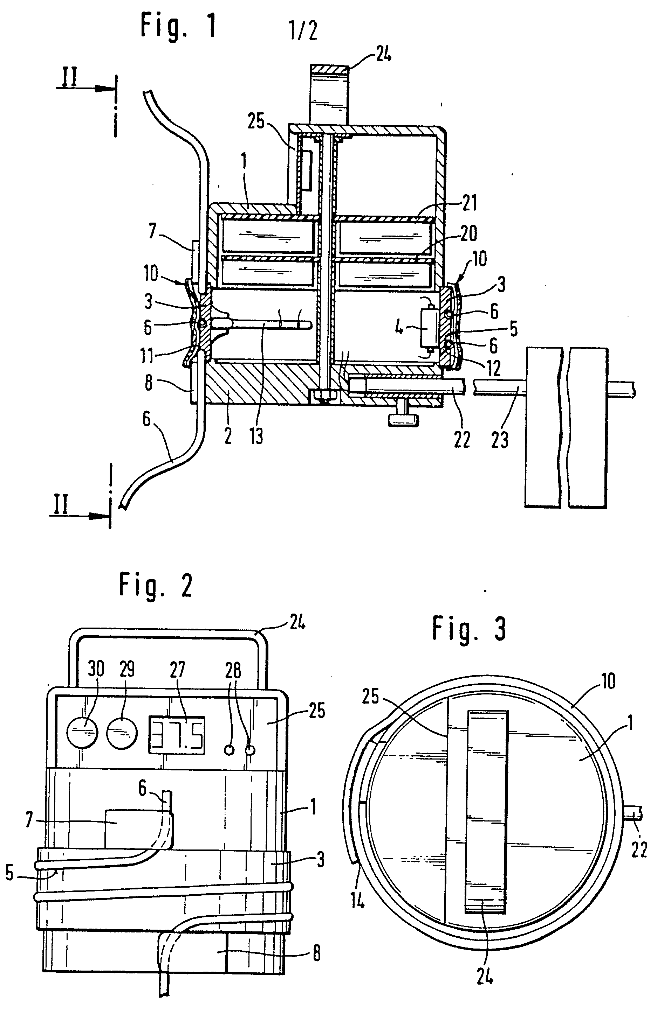

Das in Fig. 1 bis 3 dargestellte Gerät zum Anwärmen von Infusions- und/oder Transfusionslösungen weist ein Gehäuse 1 auf, das mit einem Gehäusedeckel 2 verschraubt ist. Zwischen Gehäuse 1 und Gehäusedeckel 2 ist ein Wärmeaustauschzylinder 3 gehalten. Er wird durch entlang der Innenseite am Umfang vorteilhaft angeordnete Heizwiderstände 4 angewärmt. Entlang des äusseren Umfangs des Wärmeaustauschzylinders 3 ist eine Nut oder Rille 5 vorgesehen. Sie läuft mit zwei Umschlingungen schraubenlinienförmig auf der Aussenseite von oben nach unten. In sie wird der Schlauch 6, der Bestandteil eines handelsüblichen Infusionsbestecks und ca. 190 cm lang ist, eingelegt. Er verbindet z.B. eine Blutkonserve mit der Infusionskanüle. Zur Sicherung des Einlegens und der Halterung des Schlauches 6 in der Nut 5 befindet sich am Anfang der Nut eine Einführungssicherung 7 und am Ende der Nut eine Ausführungssicherung 8. Diese Sicherungen werden im wesentlichen durch Plättchen gebildet, die die Nut soweit überdecken, dass ein Schlauch an dieser Stelle nicht ohne einen gewissen Kraftaufwand herausgenommen werden kann. Der Wärmeaustauschzylinder ist beispielsweise aus Aluminium gebildet, mit einem Aussendurchmesser von 13 cm und einer Höhe von 4 cm. In der Nut 5 wird, zur Verbesserung und Gewährleistung des Wärmeübergangs, der Schlauch 6 formschlüssig mit einer gewissen Klemmwirkung gehalten. Die Nut 5 ist im Ausführungsbeispiel 3 mm tief und hat auf ihrem Grund einen Radius von 2 mm, so dass der Schlauch 6 zum Wärmeübergang über eine grösstmögliche Oberfläche Kontakt hat. Der Schlauch hat normalerweise ebenfalls einen Radius von 2 mm, also einen Aussendurchmesser von 4 mm. Er steht also über die Aussenfläche des Wärmeaustauschzylinders 3 etwas vor. Die Nut 5 ist zur Erzielung einer Klemmwirkung etwas hinterschnitten, also bei ihrer Öffnung in die Aussenfläche des Wärmeaustauschzylinders nur 3,7 oder 3,8 mm breit, während ihre grösste lichte Weite 4 mm ist.

Zum Schutz vor Wärmeabstrahlung ist der Wärmeaustauschzylinder – bei eingelegtem Schlauch 6 – mit einer wärmeisolierten Maschette 10 umgeben. Sie ist flexibel, vorzugsweise aus textilem Material oder entsprechendem Kunststoffgewebe. Nach Einlegen des Schlauchs 6 in die Nut 5 wird die Manschette 10 auf der Aussenseite des Wärmeaustauschzylinders 3 aufgebracht. Sie ist dazu mit einem Klettenverschluss 14 versehen. Dadurch ist gewährleistet, dass sie mit einer gewissen Kraft auf den Schlauch angedrückt wird. Die Manschette 10 ist innen mit einer ziehharmonikaähnlich gefalteten Aluminiumfolie 11 ausgekleidet, die dazu beiträgt, die Wärmeabstrahlung nach aussen zu verhindern und ferner eine Wärmeübertragung von dem Wärmeaustauschzylinder 3 auf den Teil des Schlauches 6 bewirkt, der nicht direkt mit der Nut 5 Kontakt hat. Das ist also der Teil des Schlauches 6, der über die Oberfläche des Wärmeaustauschzylinders 3 ca. 1 mm hervorsteht. Auf diese Weise ist gewährleistet, dass der Schlauch 6 entlang der zwei Umschlingungen vollkommen gleichmässig allseits mit einer bestimmten Temperatur erwärmt wird.

Jedem Heizwiderstand 4 ist ein Temperaturfühler 12 zugeordnet. Der Temperaturfühler 12 leitet die Eingangs-Messgrösse für die Heizungsregelungseinheiten ab.

Ferner ist am inneren Umfang des Wärmeaustauschzylinders ein Quecksilberthermometer 13 angeordnet, das zwischen zwei Anschlüssen bei Erreichen einer bestimmten Temperatur Kontakt herstellt. Dies dient zur Sicherung des Abschaltens bei Überschreiten einer vorgegebenen Temperatur. Zur Überprüfung der Funktion ist in den Fuss des Quecksilberthermometers, mit dem er auf der Innenseite des Wärmetauschzylinders befestigt ist, eine weitere extern, also separat, ansteuerbare Heizung, eingebaut. Wird sie betätigt, z.B. über eine Prüftaste, so muss die Abschaltung stattfinden und als Störfall angezeigt werden.

Im oberen Bereich des Gehäuses befinden sich die Platinen 20 und 21 mit den Komponenten der elektrischen Schaltung. Die Anschlussleitung für die Stromversorgung der elektrischen Schaltung ist mit 22 bezeichnet. Am Gehäusedeckel 2 ist ein Bügel 23 angebracht, der zu einer Aufhängevorrichtung mit Trafo und 12 V-Leitung gehört, mit der das gesamte Gerät an einem Ständer neben einem Krankenbett aufgestellt oder aber in einem Krankenwagen neben der Trage befestigt werden kann. Auf der Oberseite des Gerätes befindet sich ein Handgriff 24. Ferner befindet sich an der Oberseite das Bedienungs- und Anzeigetableau 25 mit digitaler Temperaturanzeige 27, Kontrolllampen 28 für Heizwiderstände, zwei kombinierte Start- und Störtasten 29 und 30.

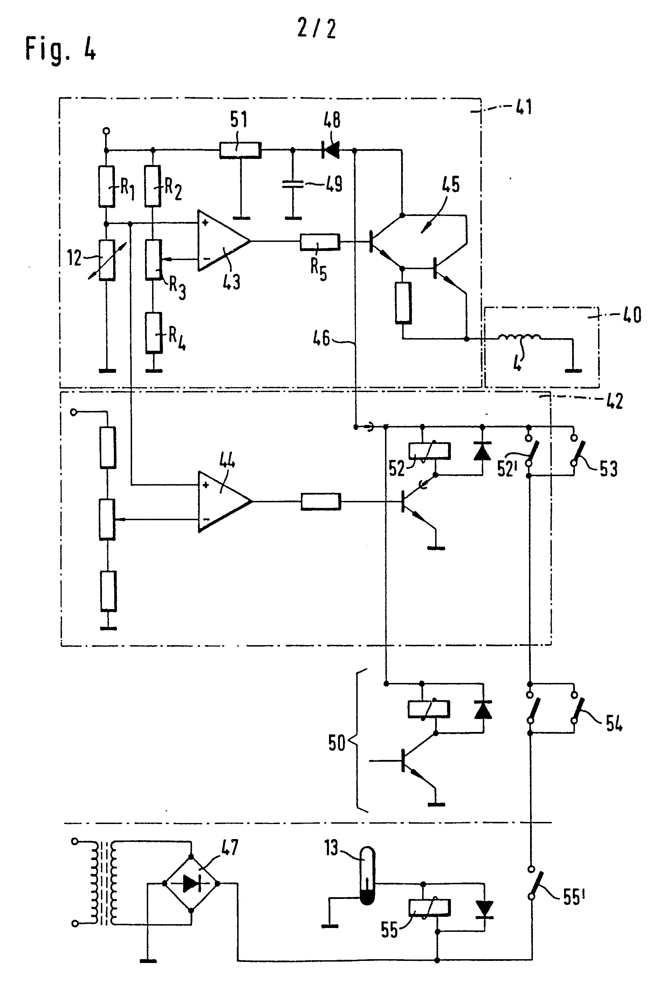

Fig. 4 zeigt den Schaltplan für zwei Heizeinrichtungen, die jeweils durch einen Heizwiderstand gebildet werden. Die Heizeinrichtung 40 wird durch den Heizwiderstand 4 gebildet (z.B. 22 Ohm). Dieser Heizeinrichtung 40 ist eine Regelschaltung 41 und eine Sicherheitsabschaltung 42 zugeordnet. Die Heizeinrichtung 40, die Regelschaltung 41 und Sicherheitsabschaltung 42 bilden zusammen eine erste Heizungsregelungseinheit. Ferner ist eine zweite Heizungsregelungseinheit 50 vorgesehen. Sie ist identisch aufgebaut.

Die Heizwiderstände 4 der zwei Heizungsregelungseinheiten 40, 41, 42 und 50 sind entlang der Innenfläche des Wärmeaustauschzylinders 3 gleichmässig verteilt. Sie sind nämlich so dimensioniert bzw. in ihrem Arbeitspunkt abgestimmt, dass sie zusammen die benötigte Heizleistung abgeben. Sie sind unabhängig voneinander derart geregelt, dass sie eine bestimmte Temperatur (z.B. 37 °C) gleichmässig halten. Dass zwei voneinander unabhängig auf eine bestimmte Temperatur eingestellte Heizungsregelungseinheiten vorgesehen sind, bringt einen Sicherungseffekt: Arbeitet eine der beiden Heizungsregelungseinheiten fehlerhaft und zwar derart, dass sie sich auf eine zu hohe Temperatur einstellt, so heizt die andere Heizungsregelungseinheit entsprechend weniger.

Die Funktionsweise der Heizungsregelungseinheit ist folgende: Der Temperaturfühler 12 wird durch einen NTC-Widerstand gebildet. Ein derartiger Widerstand ist dadurch gekennzeichnet, dass sein Widerstand mit steigender Temperatur fällt bzw. mit fallender Temperatur steigt (NTC = negative temperature controlled). Der Temperaturfühler 12 bildet mit einem weiteren Widerstand $R_1$ den ersten Zweig einer Messbrücke. Der Mittelabgriff geht an den Plus-Eingang der Verstärker 43 und 44. Der zweite Zweig der Messbrücke wird durch die Widerstände $R_2$, $R_3$ und $R_4$ gebildet, wobei der Abgriff am Widerstand $R_3$ einstellbar ist, um die Temperatur einzustellen. Der Ausgang des Verstärkers 43 geht über einen Widerstand $R_5$ an die Basis einer durch zwei Transistoren gebildeten Darlington-Schaltung 45. Die Spannungsverstärkung ist praktisch unendlich, führt also schon bei kleinsten Eingangswerten zu einem normierten Ausgangswert. Sobald also die Brückenspannung grösser als Null ist (Temperatur kleiner als die vorgegebene Bezugstemperatur), schaltet die Heizung 40 ein und es fliesst ein Heizstrom durch den Heizwiderstand 4.

Die Spannungsversorgung der Messbrücke erfolgt über die Leitung 46 vom Ausgang der Gleichrichter-Schaltung 47 her; durch die Kombination einer Diode 48, einer Kapazität 49 und einem Spannungsregler 51 erfolgt dabei eine Glättung und Stabilisierung.

Der Mittelabgriff des durch $R_1$ und dem Temperaturfühler 12 gebildeten ersten Zweiges der Messbrücke wird auch an den Plus-Eingang des Verstärkers 44 geführt. Der Minus-Eingang des Verstärkers 44 liegt wieder an einem einstellbaren festen Punkt eines weiteren Zweiges einer zweiten Brückenschaltung, deren Differenzspannung den Verstärker 44 und einen weiteren Widerstand an die Basis eines durch einen Transistor gebildeten Verstärkers gelangt. Dieser liegt im Stromkreis einen Relais 52. Erreicht also die am Temperaturfühler 12 wirksame Temperatur den vorgegebenen eingestellten Wert, dann fällt die entstehende Spannung. Der Operationsverstärker 44 geht auf Null. Der dem Ausgang des Operationsverstärkers 44 nachgeschaltete Transistor und mit ihm das Relais 52 wird stromlos; der Arbeitskontakt 51' fällt ab. Damit wird die Stromversorgung der gesamten Heizung unterbrochen. Dieser Zustand kann nur durch Betätigen der beiden Tasten 43 und 44 wieder hergestellt werden.

Beim Einschalten müssen die Tasten 53 und 54 der Heizungsregelungseinheiten 40, 41, 42, sowie 50 betätigt werden. Dann erhält die Leitung 46 Strom und das Gerät beginnt zu arbeiten.

Eine weitere Sicherheitsschaltung wird durch das Quecksilberthermometer 13 gewährleistet. Wird eine vorgegebene einstellbare Temperatur überschritten, ergibt sich Kontakt; das Relais 55 zieht an. Der Ruhekontakt 55' fällt ab. Es erfolgt eine Abschaltung der Spannungsversorgung für die Heizungsregelungseinheiten.

Es ist somit bei der Erfindung allergrössten Wert darauf gelegt, mit absoluter Sicherheit zu vermeiden, dass die Temperatur einer Infusions- oder Transfusionslösung über einen bestimmten eingestellten Wert hinaus ansteigt, da dies erhebliche Gefahren für einen Patienten mit sich bringen kann. Unabhängig voneinander arbeiten folgende Systeme:

(a) Zwei voneinander unabhängige Heizungsregelungseinheiten. Ergibt sich bei einer von ihnen, etwa im Lauf der Zeit, ein Wandern des Arbeitspunktes um ein halbes oder um ein ganzes Grad, so gleicht die andere dies entsprechend aus. Die zwei voneinander unabhängig geregelten Heizungsregelungseinheiten arbeiten also bei Temperaturveränderungen kompensierend.

(b) Jede dieser Heizungsregelungseinheiten besitzt eine Sicherheitsabschaltung, die die gesamte Spannungsversorgung unterbricht, wenn ein bestimmter einstellbarer Temperaturwert überschritten wird.

(c) Eine weitere unabhängige Sicherheitsmassnahme besteht in der durch das Quecksilberthermometer 13 und das Relais 55 gebildeten weiteren Sicherungsschaltung, die ebenfalls die Spannungsversorgung abschaltet, wenn die Temperatur der Wärmeaustauschmanschette 3 einen bestimmten Wert übersteigt.

## Patentansprüche

1. Gerät zum Anwärmen von Infusions- und Transfusionslösungen, bei dem die Infusions- oder Transfusionslösung durch einen Schlauch (6) geführt wird, der in eine Nut (5) eingelegt ist, die schraubenlinienförmig auf der Aussenseite eines mittels Heiz- und Regeleinrichtungen auf eine bestimmte Temperatur gebrachten Wärmetauschzylinders (3) vorgesehen ist, dadurch gekennzeichnet, dass der Wärmetauschzylinder (3) auf seiner Aussenseite von einer Wärmeschutzmanschette (10) umgeben ist und dass dem Gerät mindestens eine Sicherheitsabschaltung (42) zugeordnet ist, die bei Überschreiten eines bestimmten einstellbaren Temperaturwertes des Wärmetauschzylinders (3) die Heizung abschaltet.

2. Gerät nach Anspruch 1, dadurch gekennzeichnet, dass die Wärmeschutzmanschette (10) innen mit einer Aluminiumfolie (11) bedeckt ist.

3. Gerät nach Anspruch 1 oder 2, dadurch gekennzeichnet, dass die Aussenseite des Schlauches (6) über die Aussenfläche des Wärmetauschzylinders übersteht.

4. Gerät nach einem der Ansprüche 1 bis 3, dadurch gekennzeichnet, dass die Nut (5) an ihrer Öffnung in die Aussenfläche des Wärmetauschzylinders (3) zur Erzielung einer Klemmwirkung etwas hinterschnitten ist.

5. Gerät nach einem der Ansprüche 1 bis 4, dadurch gekennzeichnet, dass die Nut (5) auf der Aussenseite des Wärmetauschzylinders (3) mit mindestens einer schraubenlinienförmigen Umschlingung verläuft.

6. Gerät nach Anspruch 5, dadurch gekennzeichnet, dass die Nut (5) auf der Aussenseite des Wärmetauschzylinders (3) mit eineinhalb bis zweieinhalb schraubenlinienförmigen Umschlingungen, vorzugsweise mit zwei Umschlingungen verläuft.

7. Gerät nach einem der Ansprüche 1 bis 6, dadurch gekennzeichnet, dass der Aussendurchmesser des Schlauches (6) 4 mm, der Aussendurchmesser des Wärmetauschzylinders 100 bis 150 mm und der Krümmungsradius der Nut (5) auf ihrem Grund ca. 2 mm beträgt, und dass ferner die Nut (5) ca. 3 mm tief ist.

8. Gerät nach einem der Ansprüche 1 bis 7, dadurch gekennzeichnet, dass am Beginn der Nut (5) eine Einführungssicherung (7) und am Ende der Nut (5) eine Ausführungssicherung (8) für den Schlauch (6) vorgesehen sind, die beide (7, 8) durch ein die Nut (5) teilweise überdeckendes Plättchen gebildet werden.

9. Gerät nach einem der Ansprüche 1 bis 8, dadurch gekennzeichnet, dass die Heizeinrichtung mehrere unabhängig voneinander auf einen bestimmten Wert einstellbare temperaturregelbare Heizungsregelungseinheiten (40, 41, 42; 50) aufweist.

10. Gerät nach Anspruch 9, dadurch gekennzeichnet, dass jede Heizungsregelungseinheit (40, 41, 42; 50) zur Ableitung der Temperatur des Wärmetauschzylinders (3) als Eingangs-Messgrösse einen Temperaturfühler (12) aufweist, der durch einen negativ temperaturabhängigen Widerstand gebildet ist und der in einer Bohrung unmittelbar hinter der Nut (5) eingeklebt ist.

11. Gerät nach Anspruch 9, dadurch gekennzeichnet, dass an dem Wärmetauschzylinder (3) ein Quecksilberthermometer (13) vorgesehen ist, das beim Überschreiten eines vorgegebenen Temperaturwertes über ein Relais (54) die Abschaltung von einzelnen Heizungsregelungseinheiten bewirkt.

12. Gerät nach Anspruch 11, dadurch gekennzeichnet, dass zur Überprüfung des Quecksilberthermometers in unmittelbarer Nähe desselben eine separat ansteuerbare Heizung vorgesehen ist.

13. Gerät nach Anspruch 2, dadurch gekennzeichnet, dass die Wärmeschutzmanschette (10) mit einem Klettenverschluss (14) versehen ist.

## Revendications

1. Dispositif pour rechauffer des solutions d'infusion et de transfusion, lesquelles seront conduites par un tuyau (6) placé dans une rainure (5) en hélice sur le côté extérieur d'un système régulateur de chaleur qui apporte une chaleur définie au cylindre échangeur de chaleur (3), caractérisé par le fait que le cylindre échangeur de chaleur (3) est entouré d'un toile calorifuge (10) sur son côté extérieur et que le dispositif est muni d'un système de déconnexion (42) permettant lorsque le niveau de chaleur du cylindre de chaleur (3) programmé est dépassé de couper la chaleur.

2. Dispositif selon la revendication 1, caractérisé par le fait que la toile calorifuge (10) est recouverte a l'interieur d'une feuille d'aluminium (11).

3. Dispositif selon la revendication 1 ou 2, caractérisé par le fait que le côté extérieur du tuyau (6) dépasse sur le côté extérieur du cylindre échangeur de chaleur.

4. Dispositif d'après une des revendications de 1 à 3 caractérisé par le fait que la rainure (5) a été un peu coupée vers l'arrière de son ouverture sur la surface extérieure du cylindre échangeur de chaleur (3) pour produire un effet de serrage.

5. Dispositif d'après une des revendications de 1 à 4, caractérisé par le fait que la rainure (5) sur le côté extérieur du cylindre échangeur de chaleur (3) court avec au moins un contact en hélice.

6. Dispositif d'après la revendication 5, caractérisé par le fait que la rainure (5) court sur le côté extérieur du cylindre échangeur de chaleur (3) avec un contact en hélice d'1,5 jusqu'à 2,5, préférablement avec deux contacts.

7. Dispositif d'après une des revendications de 1 à 6, caractérisé par le fait que le diamètre extérieur du tuyau (6) est de 4 mm, le diamètre extérieur du cylindre échangeur de chaleur de 100 à 150 mm et le rayon de courbure de la rainure (5) est de 2 mm à sa base, et profond de 3 mm à son bout.

8. Dispositif d'après une des revendications de 1 à 7, caractérisé par le fait qu'au début de la rainure (5) sont prévues pour le tuyau (6), une entrée de sécurité (7) et au bout de la rainure (5) une sortie de sécurité (8), les deux sécurités (7 et 8) sont formées par des petites plaques qui en partie recouvrent la rainure (5).

9. Dispositif d'après une des revendications de 1 à 8, caractérisé par le fait que l'installation de chauffage permet d'avoir plusieurs unités de régulation de chaleur (40, 41, 42; 50) indépendantes les unes des autres.

10. Dispositif d'après la revendication 9, caractérisé par le fait que chaque unité de régulation de chaleur (40, 41, 42; 50) conduit la température du cylindre échangeur de chaleur (3) comme mesure d'entrée d'un capteur de chaleur (12), qui est formé par une résistance de température négative dépendante et est collé dans un creux tout près d'arrière de la rainure (5).

11. Dispositif d'après la revendication 9, caractérisé par le fait qu'un thermomètre à mercure (13) est prévu sur le cylindre échangeur de chaleur (3) de manière à lorsque l'unité de température donnée est dépassée d'assurer la déconnexion des unités de mesure de chaleur à l'aide d'un relais (54).

12. Dispositif d'après la revendication 11, caractérisé par le fait qu'il été prévu un chauffage fonctionnant séparèment tout près du thermomètre à mercure de manière à pouvoir vérifier son fonctionnement.

13. Dispositif d'après la revendication 2, caractérisé par le fait que la toile calorifuge (10) est pourvue d'une fermeture à glissière (14).

## Claims

1. A device for warming up infusion- or transfusion-solutions, said infusion- or transfusion-solution flowing through a tube (6) which is laid in a groove (5) placed helically on the outside of a heat exchange cylinder (3), said heat exchange cylinder (3) is heated up to a pre-set temperature by means of heating and regulating devices, characterized in that the heat exchange cylinder (3) is coated by a heat-insulating sleeve (10) and that the device has at least one safety heat regulating unit (42), said heat regulating unit shuts off the heating device when the temperature of the heat exchange cylinder (3) exceeds a pre-set temperature.

2. A device according to claim 1, characterized in that the inside of the beat-insulating sleeve (10) is coated on the inside with aluminium foil (11).

3. A device according to claim 1 or 2, characterized in that the outside of the tube (6) projects above the exterior surface of the heat exchange cylinder.

4. A device according to one of claims 1 to 3, characterized in that the groove (5) is cut back at its opening on the surface of the heat exchange cylinder (3) in order to attain a clamping effect.

5. A device according to one of claims 1 to 4, characterized in that the groove (5) runs on the outside of the heat exchange cylinder (3) with at least one helical contact.

6. A device according to claim 5, characterized in that the groove (5) runs on the outside of the heat exchange cylinder (3) with one and a half to two and a half helical contacts, preferably two contacts.

7. A device according to one of claims 1 to 6, characterized in that the external diameter of the tube (6) measures 4 mm, the external diameter of the heat exchange cylinder measures 100 to 150 mm and that the radius of curvature of the groove (5) measures about 2 mm at its base and furthermore that the groove (5) is about 3 mm deep.

8. A device according to one of claims 1 to 7, characterized in that an insertion safety clamp (7) is provided at the beginning of the groove (5) and an exit safety clamp (8) is provided at the end of the groove (5) for the tube (6), both (7, 8) are formed by small plates which partially cover the groove (5).

9. A device according to one of claims 1 to 8, characterized in that the heating device has several heat regulating units (40, 41, 42; 50) that can be ajusted to a specific value independently of one another.

10. A device according to claim 9, characterized in order to carry away the temperature from the heat echange cylinder (3) as a measured quantity of input, each heat regulating unit (40, 41, 42, 50) shows a temperature sensor (12), which is formed by a negative temperature dependent resistor, which is glued directly behind the groove (5) in a drill-hole.

11. A device according to claim 9, characterized in that a mercury thermometer (13) which effects the shut off of the heat regulating unit via a relay (54), when exceeding a pre-set temperature, is provided to the heat exchange cylinder (3).

12. A device according to claim 11, characterized in that a heating unit that can be actuated separately is provided in the immediate vicinity of the mercury thermometer in order to check it.

13. A decive according to claim 2, characterized in that the heat insulating sleeve (10) is provided with a bur fastener (14).

**Fig. 1**   1/2

**Fig. 2**

**Fig. 3**

# Fig. 4